(19) Europäisches Patentamt
European Patent Office
Office européen des brevets

(11) Veröffentlichungsnummer: **0 001 797**
**B1**

(12) **EUROPÄISCHE PATENTSCHRIFT**

(45) Veröffentlichungstag der Patentschrift: **17.06.81**

(51) Int. Cl.³: **C 12 P 35/06,**
**C 07 D 501/12**

(21) Anmeldenummer: **78101206.7**

(22) Anmeldetag: **24.10.78**

# (54) Verfahren zur Gewinnung von Cephalosporin C, seinen Salzen und Derivaten aus Kulturfiltraten oder -lösungen.

(30) Priorität: **29.10.77 DE 2748659**

(43) Veröffentlichungstag der Anmeldung:
**16.05.79 Patentblatt 79/10**

(45) Bekanntmachung des Hinweises auf die Patenterteilung:
**17.06.81 Patentblatt 81/24**

(84) Benannte Vertragsstaaten:
**BE CH DE FR GB NL**

(56) Entgegenhaltungen:
**DE - A - 1 966 849**
**US - A - 3 234 233**

(73) Patentinhaber: **HOECHST AKTIENGESELLSCHAFT**
**Postfach 80 03 20**
**D-6230 Frankfurt/Main 80 (DE)**

(72) Erfinder: **Vértesy, Laszlo, Dr.**
**Eppenhainer Weg 6**
**D-6239 Eppstein/Taunus (DE)**
Erfinder: **Huber, Gerhard, Dr.**
**In den Bleichwiesen 2**
**D-6233 Kelkheim Taunus (DE)**

**EP 0 001 797 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Gewinnung von Cephalosporin C, seinen Salzen und Derivaten aus Kulturfiltraten oder -lösungen

Die vorliegende Erfindung betrifft ein neues Verfahren zur Gewinnung von Cephalosporin C, seinen Salzen und Derivaten aus Kulturfiltraten oder -lösungen durch Einwirkung von gegebenenfalls wasserhaltigen, von Wasser verschiedenen Lösungsmitteln auf die zur Trockne gebrachten Kulturmedien und gegebenenfalls nachfolgende chemische Umsetzungen.

Cephalosporin C stellt ein wertvolles Antibiotikum dar, aus dem sich durch chemische Umsetzung die verschiedensten Arnzneimittel herstellen lassen. Es wird durch Züchtung spezieller Mikroorganismen, wie z.B. Cephalosporium acremonium, gebildet.

In den Kulturflüssigkeiten dieser Mikroorganismen befindet sich das Cephalosporin C neben vielen anderen Stoffwechselprodukten. In der Regel ist es erforderlich, das Cephalosporin C vor seiner weiteren Anwendung aus dem Fermentationsgemisch zu isolieren. Hierzu ist eine Anzahl von Verfahren beschrieben worden. So kann man beispielsweise Cephalosporin C durch Ionenaustauscher (vgl. z.B. DOS 1 492 049) oder durch Fällung mit Schwermetallsalzen (vgl. z.B. DOS 1 966 849) reinigen. Es ist weiterhin möglich, das relativ polare zwitterionische und deshalb durch mit Wasser nicht mischbare Lösungsmittel nicht extrahierbare Antibiotikum durch chemische Umsetzung in eine unpolare Form zu bringen. Dadurch wird das Cephalosporin C mit einigen mit Wasser nicht mischbaren Lösungsmitteln, wie z.B. Butanol oder Methylisobutylketon, aus wäßriger Lösung extrahierbar und kann so isoliert werden. Als hierfür geeignete Reaktionen wurden unter anderem Umsetzungen der freien Aminogruppe und der freien Carboxylgruppen des Cephalosporin C vorgeschlagen (vgl. beispielsweise US-Patent 3 234 223). Alle diese Verfahren verlaufen nur dann befriedigend, wenn die wäßrigen Lösungen reines Cephalosporin C enthalten. Die aus den Fermentationslösungen gewonnenen Filtrate enthalten jedoch eine Vielzahl von Verunreinigungen, wie z.B. Fette, Salze, Eiweißstoffe oder Polysaccharide, die die geschilderten Reinigungsverfahren erheblich stören. So verlaufen z.B. die Umsetzungen des Cephalosporin C zu extrahierbaren Derivaten nur mit Ausbeuten unter 50%, während unter gleichen Bedingungen mit wäßrigen Lösungen von reinem Cephalosporin C über 90%ige Umsetzungen erzielt werden. Deshalb ist von verschiedenen Seiten vorgeschlagen worden, vor die genannten Trennoperationen eine wirksame Vorreinigung vorzuschalten (vgl. z.B. J. Appl, Chem. Biotechn. *26* (1976), Seiten 459 bis 468). So eine Vorreinigung kann darin bestehen, daß das Kulturfiltrat mit der 2- bis 3-fachen Menge Aceton versetzt wird. Hierdurch wird ein Teil der Verunreinigungen unlöslich und kann durch Filtration entfernt werden. Da hierfür jedoch bei in der Technik üblichen Großfermentationen sehr große Mengen Aceton benötigt werden, steht dies einer praktischen Durchführung entgegen. Ungünstig ist weiterhin, daß mit den Verunreinigungen ein Teil des Cephalosporin C mitgerissen wird, was die Ausbeute verringert.

Eine andere Vorreinigung besteht in der Verwendung von Adsorptionsharzen, wie sie z.B. in der DOS 2 021 696 beschrieben ist. Da hierbei jedoch beträchtliche Mengen eines teuren Harzes eingesetzt werden müssen und zu seiner Behandlung große Volumina von Wasser und von wäßrigen organischen Lösungsmitteln erforderlich sind, die ein Mehrfaches des Fermentationsvolumens ausmachen können, stellt die Anwendung derartiger Harze auch keine optimale Lösung dar. Als weiterer ungünstiger Faktor kommt noch ein erheblicher apparativer Aufwand hinzu.

Das erfindungsgemäße Verfahren umgeht diese Schwierigkeiten in der Weise, daß das Kulturfiltrat oder die Kulturlösung, in dem (der) das gewünschte Antibiotikum enthalten ist, zunächst getrocknet und danach das Trockenprodukt in einem gegebenenfalls wasserhaltigen, von Wasser verschiedenen Lösungsmitteln aufgenommen wird. Im Hinblick auf den Stand der Technik war es überraschend, daß sich in den so erhaltenen Lösungen chemische Umsetzungen des Cephalosporin C zu Derivaten oder unlöslichen Salzen mit sehr guten Ausbeuten durchführen lassen. Die so erhaltenen Derivate können anschließend entweder direkt aus dem organischen Lösungsmittel ausgefällt oder zwischen Wasser und einem mit Wasser nicht mischbaren Lösungsmittel verteilt und aus der abgetrennten organischen Phase mit einem geeigneten Zusatz, beispielsweise in besonders vorteilhafter Form einem Diamin, gefällt werden. Hat man in dem von Wasser verschiedenen Lösungsmittel zur Isolierung ein unlösliches Salz gebildet, so kann man dieses leicht abfiltrieren.

Erfindungsgemäß wurde somit ein Verfahren zur Gewinnung von Cephalosporin C, seinen Salzen und Derivaten aus Kulturfiltraten oder -lösungen entwickelt, das dadurch gekennzeichnet ist, daß man das Kulturfiltrat oder die Kulturlösung trocknet, in einem gegebenenfalls wasserhaltigen, von Wasser verschiedenen Lösungsmittel aufnimmt und

a) ein Derivat der Aminogruppe des Cephalosporin C bildet, das keine Zwitterionenstruktur mehr aufweist und dieses als freie Säure oder als Salz isoliert oder

b) das Cephalosporin C direkt oder als Schwerlösliches Salz aus dem von Wasser verschiedenen Lösungsmittel ausfällt.

Die Trocknung des Kulturfiltrats bzw. der Kulturlösung kann unter Anwendung an sich bekannter Verfahren erfolgen. So eignen sich zum Beispiel die Gefriertrocknung, Sprühtrocknung oder auch destillative Verfahren, insbesondere die Vakuumdestillation, wobei berücksichtigt werden muß, daß die angewandten Temperaturen nicht zu hoch liegen, um eine vollstandige oder teilweise Inaktivierung des temperaturempfindlichen Antibiotikums zu verhindern. Wegen der einfachen und preiswerten Durchführbarkeit wird man erfindungsgemäß der Sprühtrocknung den Vorzug geben. Die Kulturlösung oder das Kulturfiltrat können direkt oder auch nach vorheriger Änderung des pH-Wertes getrocknet werden. Eine Einstellung des pH-Wertes kann insbesondere dann zweckmäßig sein, wenn durch mäßiges Ansäuern ein Teil der Verunreinigungen bereits ausgefällt werden kann. Die zu trocknende Flüssigkeit sollte aber so beschaffen sein, daß bei der entsprechenden Konzentrierung keine extremen pH-Werte entstehen, wie z.B. pH-Werte unter 2 oder über 9. Vorzugsweise wird man bei pH-Werten zwischen etwa 4 und etwa 7 arbeiten. Die Trocknungsverfahren führen zu Produkten, die noch eine gewisse Restfeuchte haben können. Diese kann in starkem maße variieren, in der Regel zwischen etwa 0,1 und 50%. Der sich üblicherweise ergebende Wert liegt im allgemeinen zwischen etwa 2 und 12%.

Die Extraktion des Cephalosporin C aus dem getrockneten Kulturfiltrat bzw. der getrockneten Kulturlösung kann im Prinzip mit jedem von Wasser verschiedenen Lösungsmittel vorgenommen werden, in dem das Cephalosporin ausreichend löslich ist. Wird ein solches Lösungsmittel ohne Zusatz von Wasser angewandt, so kommen beispielsweise Formamid oder Eisessig in Betracht.

Es ist nicht unbedingt erforderlich, daß bei der Extraktion wasserfrei gearbeitet werden muß. Ein geringer Wassergehalt, der in Abhängigkeit von dem verwendeten Lösungsmittel vorzugsweise einige %, wie beispielsweise etwa 3 bis 30% betragen kann, erhöht das Lösungsvermögen vieler Lösungsmittel so weit, daß sie ebenfalls zur Extraktion des Cephalosporin C herangezogen werden können. Wählt man aber einen zu hohen Wasseranteil, beispielsweise über 30%, so verringert sich das erfindungsgemäße gute Reaktionsverhalten des Cephalosporin C. Als derartige Lösungsmittel, die vorzugsweise mit einem bestimmten Wassergehalt zur Anwendung kommen, seien beispielsweise genannt Acetonitril, Dimethylformamid, niedrig molekulare aliphatische Alkohole, vorzugsweise Methanol, Methylcellosolve, Phenol, Pyridin oder Trimethylphosphat sowie Mischungen dieser Lösungsmittel untereinander. Es ist selbsverständlich auch möglich, diese Lösungsmittel mit den üblicherweise in wasserfreier Form eingesetzten Lösungsmitteln, wie z.B. Eisessig oder Formamid zu mischen oder mit anderen, die Lösungsfähigkeit steigernden Zusätzen zu versehen. Beim Einsatz wasserhaltiger Lösungsmittel hat sich besonders Methanol mit einem Wassergehalt von etwa 5 bis etwa 30%, insbesondere 5 bis 15%, vorzugsweise von 10% bewährt.

Bei der Extraktion des trockenen Kulturfiltrats oder der trockenen Kulturlösung ist es möglich, daß die gesamte Trockenmasse oder auch nur ein Teil davon in das Lösungsmittel aufgenommen wird. Wesentlich ist, daß das angewandte Lösungsmittel optimale Lösungseigenschaften für Cephalosporin C besitzt. Der Umfang, in dem die Trockenmasse in Lösung geht, hängt in erster Linie von dem eingesetzten Nährboden ab. Die erfindungsgemäße gute Reaktionsfähigkeit der durch die Extraktion entstehenden Cephalosporin C-Lösungen wird davon nicht beeinträchtigt.

Die weitere Isolierung des Cephalosporin C aus den Trockensubstanz-Extrakten kann nunmehr auf verschiedene Weise erfolgen.

So ist es möglich, das Cephalosporin C aus der erhaltenen Lösung durch eine Direktfällung zu gewinnen. Während aus wäßrigen Lösungen das Cephalosporin nur schwierig als Niederschlag ausscheidbar ist, gelingt eine solche Fällung in den erfindungsgemäß eingesetzten, von Wasser verschiedenen Lösungsmitteln.

Für eine Fällung besonders geeignet sind beispielsweise anorganische Ionen von Schwermetallen, die mit dem Cephalosporin C ein unlösliches Salz bilden, wie z.B. zweiwertige Kobalt-, Kupfer-, Zinn- oder Zink-Ionen, organische Basen, wie z.B. Acridin oder Rivanol oder polare Lösungsmittel, wie z.B. Aceton, niedrig molekulare Alkohole, insbesondere mit 3 bis 6 Kohlenstoffatomen, wie z.B. n-Propanol, Isopropanol, Butanole, wie z.B. n-Butanol, Isobutanol, Amylalkohol oder Äthylacetat. Sind die Lösungen stark verunreinigt, d.h., beträgt der Feststoffgehalt etwa 8% und mehr, so kann es vorkommen, daß bei einer Fällung der Niederschlag noch verunreinigt ist. In solchen Fällen ist es zweckmäßig, eine Vorfällung durchzuführen, wobei ein Großteil der Verunreinigungen aus der Lösung ausgeschieden wird, nicht jedoch das Cephalosporin C. Solche Vorfällungen sind mit an sich bekannten Fällungsreagenzien durchführbar, wie z.B. mit Tannin, Phosphorwolframsäure, Flaviansäure, Kalium-wismutjodid, oder Rivanol. Aus dem Überstand der Vorfällung gelingt dann die Abtrennung des Cephalosporin nahezu vollständig mit einem der vorstehend erwähnten Fällungsmittel, wie beispielsweise mit Zinkacetat.

Eine andere Möglichkeit, das Cephalosporin aus dem Extrakt der trockenen Kulturlösung bzw. des trockenen Kulturfiltrats zu isolieren, besteht darin, daß in den zur Extraktion erfindungsgemäß verwendeten, von Wasser verschiedenen Lösungsmitteln leicht und unproblematisch Umsetzungen der freien Aminogruppe des Cephalosporin C zu an sich bekannten Derivaten durchgeführt werden können. Es entstehen hierbei Verbindungen, die keine Zwitterionenstruktur mehr aufweisen.

Besonders leicht gelingen so Umsetzungen

der Aminogruppe zu Acylderivaten mit Säurehalogeniden, vorzugsweise Säurrechloriden, wie beispielsweise mit Benzoylchlorid oder mit Chlorameisensäurebenzylester. Die Umsetzung kann auf die für diese Reaktionen an sich bekannte Weise durchgeführt werden. Es können auch andere Säurehalogenide, vorzugsweise Chloride zur Anwendung kommen, z.B. von gegebenenfalls substituierten aliphatischen Carbonsäuren mit $C_2$ bis $C_{18}$ wie z.B. Acetylchlorid, Acetylbromid, Chloracetylchlorid, Propionylchlorid, n-Butyrylchlorid, n-Valerylchlorid, Isovalerylchlorid, Caprylchlorid, n-Laurylchlorid, n-Palmitylchlorid, n-Stearylchlorid, von gegebenenfalls substituierten aromatischen Carbonsäuren, wie z.B. Benzoylbromid, Mon-, Di- oder Trichlorbenzoylchloride, Mono-, Di- oder Trinitrobenzoylchloride, gegebenenfalls substituierte Sulfonsäurehalogenide, wie z.B. Benzoylsulfonylchlorid, seine Alkylderivate, wie z.B. Toluolsulfonylchlorid, sowie Naphthylsulfonylchloride und seine Derivate. Will man unter schonenden Bedingungen arbeiten, so können in entsprechenden Lösungsmitteln auch die etwas reaktionsträgeren Säureanhydride als Reaktionspartner in an sich bekannter Weise als Acylierungsreagenzien eingesetzt werden wie z.B. Acetanhydrid. Auch hier ist es möglich, andere aliphatische oder aromatische Säureanhydride zu verwenden, beispielweise Säureanhydride der Fettsäuren mit 2 bis 18 C-Atomen, wie z.B. Propionsäure, Buttersäure, n-Valeriansäure, Isovaleriansäure, n-Capronsäure oder Isocapronsäure, Säureanhydride der aromatischen Carbonsäuren, wie z.B. der Benzoesäure, Phenylessigsäure, Phenylpropionsäure, Zimtsäure, sowie von deren Halogen-, Nitro- und Alkylderivaten, wie z.B. der Chlor-, Dichlor- oder Trichlorbenzoesäure, der Mono- und Dinitrobenzoesäure, sowie der Methyl-, Äthyl-, Propyl-, n-Butyl- oder Isobutyl-Derivate der genannten aromatischen Carbonsäuren. Auch gemischte Anhydride der genannten Carbonsäuren können erfindungsgemaß eingesetzt werden.

Erfindungsgemäß ist es besonders vorteilhaft, wenn bei Reaktionen dieser Art ein Acetylierungsreagenz eingesetzt wird. Eine Acetylierung hat sich als besonders günstig erwiesen, da sich in den Kulturfiltraten neben Cephalosporin C noch dessen Desacetylderivat, das Desacetylcephalosporin C befindet, das in 3-Stellung anstelle einer Acetoxygruppe eine Hydroxylgruppe besitzt. Die Desacetylverbindung ist mikrobiologisch weit weniger wirksam als Cephalosporin C und ihr Vorkommen ist daher in Cephalosporin C-Fermentationen wenig erwünscht, aber nicht zu umgehen. Bei einer erfindungsgemäß durchgeführten Acetylierung wird nun nicht nur die Aminogruppe acetyliert, sondern auch die genannte Hydroxylgruppe, wodurch aus beiden Verbindungen, dem Cephalosporin C und seinem Desacetylderivat, das gleiche Reaktionsprodukt entsteht. Eine solche, die Ausbeute günstig beeinflussende doppelte Umsetzung kann beispielsweise in Eisessig als Lösungsmittel durchgeführt werden. Für die Acetylierung können an sich bekannte Acetylierfungsreagenzien, wie z.B. Acetylchlorid, Essigsäureanhydrid, Keten oder andere eingesetzt werden. Vorteilhaft ist es, Katalysatoren, wie z.B. Pyridin oder Triäthylamin für die in an sich bekannter Weise durchzuführende Acetylierungsreaktion zu verwenden.

Die Umsetzung des Cephalosporin C mit anderen Aminreagenzien ist unter den erfindungsgemäßen Bedingungen ebenfalls erleichtert. So gelingen Umsetzungen mit Isocyanaten, wie z.B. mit Phenylisocyanat oder Naphthylisocyanat und Isothiocyanaten, z.B. mit Phenylisothiocyanat in guter Ausbeute zu den entsprechenden Harnstoff- bzw. Thioharnstoffderivaten, die sich in der Regel leicht kristallisieren lassen.

Auch die Bildung von Schiffchen basen mit Aldehyden, wie z.B. mit 5-Chlorsalicylaldehyd, als Aminreagenzien ist erfindungsgemäß gut möglich. Vorteilhaft ist weiterhin die Umsetzung mit o-Nitrophenylsulfenylchlorid (NPS-Chlorid) zu dem NPS-Derivat. Dieses unpolare Umsetzungsprodukt ist aus wäßriger Lösung sogar mit dem selektiveren Lösungsmittel Äthylacetat extrahierbar und somit leichter rein zu gewinnen.

Die unpolaren Derivate des Cephalosporin C, deren Darstellung vorstehend dargelegt wurde, können aus einer wäßrigen Lösung durch Extraktion in an sich bekannter Weise mit einem mit Wasser nicht völlig mischbaren Lösungsmittel, wie z.B. n-Butanol, Isobutanol, Äthylacetat oder Methylisobutylketon, vorzugsweise jedoch mit n-Butanol isoliert werden. Aus der organischen Phase kann dann das cephalosporin C-Derivat in an sich bekannter Weise z.B. durch Zugabe von Chinolin oder Isochinolin als Salz ausgeschieden werden. Eine solche Chinolinsalzfällung hat jedoch den Nachteil, daß sie bei Cephalosporin C-Konzentrationen unter 1% nicht vollständig abläuft, so daß die Ausbeuten nicht besonders gut sind.

Uberraschenderweise ist erfindungsgemäß gefunden worden, daß anstelle von Chinolin oder Isochinolin sehr vorteilhaft organische Diamine eingesetzt werden können. Als Diamine können ganz allgemein unsubstituierte oder substituierte Alkylendiamine, aber auch aromatische Diamine zur Fällung verwendet werden. Als Alkylendiamine eignen sich besonders solche mit der allgemeinen Formel

$$H_2N—(CH_2)_n—NH_2$$

wobei n für eine Zahl zwischen 4 und 12, vorzugsweise zwischen 5 und 7 stehen kann, sowie deren Derivate, die beispielsweise dadurch entstehen, daß ein Wasserstoffatom an einem oder beiden Stickstoffatomen oder ein oder mehrere Wasserstoffatome der Methylengruppen durch niedrig molekulares Alkyl, vorzugsweise Methyl oder Aryl, vorzugsweise Phenyl, ersetzt sind. Als erfindungsgemäß geeignete Amine

seien beispielsweise genannt das 1,6-Diaminoheptan, 2,7-Diaminooctan, 2-Methylhexamethylendiamin, 3-Methyl-hexamethylendiamin oder N-Methyl-hexamethylendiamin, sowie andere N-Alkylderivate, z.B. N-Äthyloder N-Propylderivate oder N-Phenylderivate, sowie N,N'-Dialkylderivate, wie z.B. N,N'-Dimethyl-, N,N'-Diäthyloder N,N'-Dipropyl-hexamethylendiamin. Auch die tertiären und quartären Basen, beispielsweise des Hexamethylendiamins, sind erfindungsgemäß anwendbar. Als erfindungsgemäß einsetzbare aromatische Diamine kommen z.B. Diaminobenzole, insbesondere 1,3-Diaminobenzol und seine Derivate, vorzugsweise Methyl- und Halogen-, vorzugsweise Chlorderivate in Betracht.

Verwendet man erfindungsgemäß die Diamine, insbesondere Alkylendiamine mit 5 bis 7 C-Atomen, vorzugsweise das Hexamethlendiamin als Salzbildner, so erhält man eine selektivere, nahezu vollständige kristalline Fällung des Cephalosporin C-Derivats. Hierbei ist nicht nur die Ausbeute besser als bei der Chinolin- oder Isochinolinsalzfällung, sondern auch die Reinheit des Produktes.

Zur Isolierung der unpolaren Derivate ist es nicht unbedingt erforderlich, eine Extraktion aus wäßriger Lösung vorzunehmen. In Abhängigkeit von den Lösungseigenschaften und den unerwünschten Verunreinigungen des Kulturmediums kann auch so vorgegangen werden, daß man die unpolaren Derivate direkt als freie Säure, beispielsweise durch Einengen der Lösung oder Zugabe anderer geeigneter Lösungsmittel, wie z.B. Äther, Äthylacetat oder Butylacetat ausfällt. Es ist auch möglich, sie durch Zugabe von Fällungsmitteln auszufällen, wie z.B. mit anorganischen Ionen von Schwermetallen, die mit dem Cephalosporin C-Derivat ein unlösliches Salz bilden, wie beispielsweise zweiwertigen Kobalt-, Kupfer-, Zinn-, insbesondere Zink-Ionen, oder organischen Basen, wie beispielsweise Acridin oder Rivanol.

In der Regel ist es nicht erforderlich, für die weitere Umsetzung zu Arzneimitteln die Derivate abzuspalten oder die Salze in Freiheit zu setzen, da die bevorzugte Weiterverarbeitung im allgemeinen in einer Abspaltung des Acylrestes in an sich bekannter Weise zur 7-Aminocephalosporansäure besteht. Liegt dennoch Interesse an der Gewinnung von Cephalosporin C vor, so können die vorstehend beschriebenen Derivate in literaturbekannter Weise abgespalten oder auch die Salze beispielsweise in an sich bekannter Weise durch Behandlung mit Ionenaustauschern in die freie Säure umgewandelt werden.

Die folgenden Ausführungsbeispiele erläutern die vorstehende Erfindung, ohne sie jedoch auf sie zu beschränken.

Beispiel 1

180 l eines klaren Kulturfiltrates von einer Cephalosporium acremonium-Fermentation werden innerhalb von 6 Stunden sprühgetrocknet. Es werden 17 kg Trockenkulturfiltrat in Form eines hellbraunen Pulvers mit einer Restfeuchte von 3% erhalten. Der Cephalosporin C-Gehalt beträgt 48 g/kg. Daneben liegen noch 10 g/kg Desacetylcephalosporin C vor.

1 kg dieses Trockenkulturfiltrats word mit 3 l Eisessig versetzt und nach 10 Minuten Rühren mit einem Ultra-Turrax[(R)] abzentrifugiert. Der ungelöste Rückstand wird erneut mit 1 l Eisessig ausgerührt, abzentrifugiert und die klaren Zentrifugationsdüberstände vereinigt. Dann werden 350 ml Acetanhydrid und 200 ml Pyridin hinzugefügt und bei Raumtemperatur über Nacht stehen gelassen. Anschließend wird unter vermindertem Druck bis auf etwa 1 l eingeengt und die resultierende zähflüssige Masse mit 2 l Aceton gefällt. Im Fällungsniederschlag befindet sich das acetylierte Cephalosporin. Es wird abgetrennt, in 2 1 Methanol gelöst und aus der geklärten Methanollösung mit einer methanolischen Lösung von 100 g Zinkacetat gefällt. Die Fällung wird über Nacht stehengelassen, anschließend die feste Phase abgetrennt und getrocknet. In den 92 g des Niederschlages befinden sich 51 g Zinksalz de acetylierten Cephalosporin C.

Beispiel 2

Zu 1 kg der nach Beispiel 1 erhaltenen Trockenkulturfiltrats werden 3 l einer Mischung von Methanol-Wasser (9:1) gegeben, gründlich gerührt, filtriert und das Ungelöste erneut mit 2 l der gleichen Mischung ebenso extrahiert. Die vereinigten flüssigen Phasen (4,5 l) enthalten 10 g Cephalosporin C pro 1. 2 l dieses methanolischen Extraktes werden mit 40%iger Natronlauge auf einen scheinbaren pH-Wert von 8,6 eingestellt. Die pH-Messung erfolgt mit einem pH-Meter der Firma Knick. Anschließend werden innerhalb von 30 Minuten unter Rühren 40 ml Benzoylchlorid und 40%ige Natronlauge so zur Lösung getropft, daß der gemessene pH-Wert in den Grenzen zwischen 8 und 9 bleibt. Hierzu werden 65 ml der natronlauge benötigt. Dann wird noch einige Minuten weitergerührt, mit 2n Salzsäure auf pH 6 gestellt und im Vakuum das Methanol abgezogen. Die resultierende wäßrige Phase wird zunächst im Scheidetrichter mit Äthylacetat entfettet, dann auf pH 2,2 gestellt und zweimal mit n-Butanol extrahiert. Die klaren Butanolphasen werden im Vakuum durch Azeotrop-Destillation getrocknet und dann mit 1,6-Diaminohexan auf einen scheinbaren pH-Wert von 5 gestellt. Die spontane Kristallisation wird durch Stehen über Nacht vervollständigt, die kristalline Phase durch Abnutschen gesammelt und im Vakuum getrocknet. 22 g des hellen Niederschlags enthalten das N-Benzoyl-cephalosporin C-hexamethylendiaminsalz in 82%iger Reinheit.

Beispiel 3

2 l des nach Beispiel 2 gewonnenen methanolischen Extraktes werden, wie in Beispiel 2, auf einen pH-Wert von 8,5 eingestellt. Dann

werden 60 ml Phenylisocyanat unter Rühren langsam hinzugegeben und der scheinbare pH-Wert mit Natronlauge zwischen 8 und 9 gehalten. Nach einer Reaktionszeit von 60 Minuten wird mit 2n Salzsäure auf pH 6 gestellt, im Vakuum eingeengt, mit Äther extrahiert und die abgetrennte organische Phase verworfen. Die Wasserphase wird dann weiter auf pH 3 angesäuert und zweimal mit n-Butanol extrahiert. Die vereinigten Butanolextrakte werden im Vakuum auf 1/5 eingeengt. Dabei entsteht ein Kristallbrei, der 85% des eingesetzten Cephalosporin C enthält. Es wird auf der Filternutsche abgesaugt, mit wenig Äther gewaschen und getrocknet.

Beispiel 4

Zu 1 l der nach Beispiel 2 gewonnenen methanolischen Lösung wird 1 g Phosphorwolframsäure, gelöst in 20 ml Methanol, langsam unter Rühren hinzugegeben. Hierbei entsteht ein heller, voluminöser Niederschlag, der in einer Laboratoriumzentrifuge bei 4000 g abgeschleudert und anschliessend verworfen wird. Der klare Überstand, der 9.8 mg Cephalosporin C pro ml enthält, wird dann unter Rühren mit 300 ml einer 10%igen methanolischen Zinkacetat-Hydratlösung versetzt. Es fällt ein heller Niederschlag aus, der zur Vervollständigung der Fällung mit einigen mg kristallinem Cephalosporin C-Zink-Salz angeimpft und über Nacht bei 6°C stehengelassen wird. Danach wird der Niederschlag durch Zentrifugieren gesammelt, von der flüssigen Phase abgetrennt, mit Aceton gewaschen und im Vakuum getrocknet. Das resultierende Pulver enthält 9,5 g des Cephalosporin C als Zinksalz.

**Patentansprüche**

1. Verfahren zur Gewinnung von Cephalosporin C, seinen Salzen und Derivaten aus Kulturfiltraten oder -lösungen, dadurch gekennzeichnet, daß man das Kulturfiltrat oder die Kulturlösung trocknet, in einem gegebenenfalls wasserhaltigen, von Wasser verschiedenen Lösungsmittel aufnimmt, und

a) ein Derivat der Aminogruppe des Cephalosporin C bildet, das keine Zwitterionenstruktur mehr aufweist und dieses als freie Säure Salz isoliert oder

b) das Cephalosporin C direkt oder als Schwerlösliches Salz ausfällt.

2. Verfahren nach Anspruch 1, dadurch gekennzeichnet, daß das Derivat nach Überführung in eine wäßrige Phase und Extraktion mit einem mit Wasser nicht völlig mischbaren Lösungsmittel durch Ausfällung aus diesem isoliert wird.

3. Verfahren nach Anspruch 1, dadurch gekennzeichnet, daß die Isolierung des Derivats durch direkte Fällung aus dem gegebenenfalls wasserhaltigen, von Wasser verschiedenen Lösungsmittel erfolgt.

4. Verfahren nach Anspruch 1, dadurch gekennzeichnet, daß die Trocknung als Sprühtrocknung durchgeführt wird.

5. Verfahren nach Anspruch 1, dadurch gekennzeichnet, daß als von Wasser verschiedenes Lösungsmittel Formamid oder Eisessig eingesetzt wird.

6. Verfahren nach Anspruch 1, dadurch gekennzeichnet, daß als wasserhaltiges, von Wasser verschiedenes Lösungsmittel Acetonitril, Dimethylformamid, ein niedrigmolekularer aliphatischer Alkohol, vorzugsweise Methanol, Methylcellosolve, Phenol, Pyridin oder Trimethylphosphat verwendet wird.

7. Verfahren nach Anspruch 1, dadurch gekennzeichnet, daß die Derivatbildung mit Säurehalogeniden oder Säureanhydriden erfolgt.

8. Verfahren nach Anspruch 7, dadurch gekennzeichnet, daß als Säurehalogenide Benzoylchlorid und als Anhydride Essigsäureanhydrid eingesetzt werden.

9. Verfahren nach Anspruch 1, dadurch gekennzeichnet, daß die direkte Fällung in Form eines schwerlöslichen Schwermetallsalzes, vorzugsweise Zinksalz erfolgt.

10. Verfahren nach Anspruch 2, dadurch gekennzeichnet, daß die Fällung aus der organischen Phase durch direkte Kristallisation erfolgt.

11. Verfahren nach Anspruch 2, dadurch gekennzeichnet, daß die Fällung aus der organischen Phase als Salz mit einem Alkylendiamin oder einem aromatischen Diamin durchgeführt wird.

12. Verfahren nach Anspruch 11, dadurch gekennzeichnet, daß ein gegebenenfalls substituiertes Alkylendiamin der Formel

$$NH_2—(CH_2)_n—NH_2$$

eingesetzt wird, in der n für eine Zahl zwischen 4 und 12, vorzugsweise 5 und 7 steht.

13. Verfahren nach Anspruch 12, dadurch gekennzeichnet, daß ein Wasserstoff am Stickstoff und/oder ein oder mehrere Wasserstoffatome der Methylengruppe durch niedrigmolekulares Alkyl oder Aryl substituiert sind.

14. Verfahren nach Ansprüchen 11 bis 13, dadurch gekennzeichnet, daß die Fällung aus der organischen Phase als 1,6-Diaminohexan-Salz erfolgt.

15. Verfahren nach Anspruch 2, dadurch gekennzeichnet daß das mit Wasser nicht völlig mischbare Lösungsmittel n-Butanol, Isobutanol, Äthylacetat oder Methylisobutylketon ist.

**Revendications**

1. Procédé d'obtention de céphalosporine C, de ses sels et dérivés, à partir de filtrats ou de solutions de culture, caractérisé en ce qu'on sèche le filtrat de culture ou la solution de

culture, on reprend dans un solvant différent de l'eau, contenant éventuellement de l'eau, et

a) on forme un dérivé du groupe amino de la céphalosporine C, qui ne présente plus de structure zwitterionique, et on isole celui-ci sous forme d'acide libre ou de sel, ou

b) on précipite la céphalosporine C directement ou sous la forme d'un sel difficilement soluble.

2. Procédé selon la revendication 1, caractérisé en ce qu'après le transfert dans une phase aqueuse et l'extraction avec un solvant non totalement miscible à l'eau, le dérivé est isolé du solvant par précipitation.

3. Procédé selon la revendication 1, caractérisé en ce que l'isolement du dérivé a lieu par précipitation directe à partir d'un solvant différent de l'eau, contenant éventuellement de l'eau.

4. Procédé selon la revendication 1, caractérisé en ce que le séchage a lieu sous la forme d'un séchage par atomisation.

5. Procédé selon la revendication 1, caractérisé en ce que, comme solvant différent de l'eau, on utilise le formamide ou l'acide acétique glacial.

6. Procédé selon la revendication 1, caractérisé en ce que comme solvant différent de l'eau, contenant de l'eau, on utilise l'acétonitrile, le diméthylformamide, un alcool aliphatique inférieur, le diméthylformamide, un alcool aliphatique inférieur, de préférence le méthanol, le méthylcellusolve, le phénol, la pyridine ou le phosphate de triméthyle.

7. Procédé selon la revendication 1, caractérisé en ce que la formation du dérivé est réalisée avec des halogénures d'acides ou des anhydrides d'acides.

8. Procédé selon la revendication 7, caractérisé en ce que, comme halogénure d'acide, on utilise le chlorure de benzoyle et comme anhydride l'anhydride acétique.

9. Procédé selon la revendication 1, caractérisé en ce que la précipitation directe a lieu sous la forme d'un sel de métal lourd difficilement soluble, de préférence un sel de zinc.

10. Procédé selon la revendication 2, caractérisé en ce que la précipitation a lieu à partir de la phase organique par cristallisation directe.

11. Procédé selon la revendication 2, caractérisé en ce que la précipitation est effectuée à partir de la phase organique sous la forme d'un sel avec une alkylène diamine ou une diamine aromatique.

12. Procédé selon la revendication 11, caractérisé en ce qu'on utilise une alkylène diamine éventuellement substituée, de formule

$$NH_2—(CH_2)_n—NH_2$$

dans laquelle n représente un nombre compris entre 4 et 12 de préférence entre 5 et 7.

13. Procédé selon la revendication 12, caractérisé en ce qu'un atome d'hydrogène sur l'azote et/ou un ou plusieurs atomes d'hydrogène du groupe méthylène sont substitués par un alkyl inférieur ou un aryle.

14. Procédé selon l'une quelconque des revendications 11 à 13, caractérisé en ce que la précipitation a lieu à partir de la phase organique sous la forme d'un sel de 1,6-diaminohexane.

15. Procédé selon la revendication 2, caractérisé en ce que le solvant non totalement miscible à l'eau est le n-butanol, l'isobutanol, l'acétate d'éthyle ou la méthyl isobutyl cétone.

**Claims**

1. A process for obtaining cephalosporin C, the salts and derivatives thereof form culture filtrates or culture solutions, which comprises drying the culture filtrate or the culture solution, dissolving it in an optionally water-containing solvent different from water, and

a) forming a derivative of the amino group of cephalosporin C having no longer a hybrid ion structure, and isolating it in the form of a free acid or a salt, or

b) precipitating the cephalosporin C or the scarcely soluble salt thereof directly.

2. The process as claimed in claim 1, which comprises isolating the derivative, after conversion to an aqueous phase and extraction with a solvent not completely miscible with water, by precipitation from the latter solvent.

3. The process as claimed in claim 1, which comprises isolating the derivative by direct precipitation from the optionally water-containing solvent different from water.

4. The process as claimed in claim 1, which comprises carrying out the drying as spray-drying.

5. The process as claimed in claim 1, which comprises using formamide or glacial acetic acid, as solvent different from water.

6. The process as claimed in claim 1, which comprises using as water-containing solvent different from water acetonitrile, dimethyl formamide, a low molecular aliphatic alcohol, preferably methanol, methyl cellosolve, phenol, pyridine or trimethyl phosphate.

7. The process as claimed in claim 1, which comprises forming the derivative by means of acid halides or acid anhydrides.

8. The process as claimed in claim 7, which comprises using as acid halides benzoyl chloride, and as anhydride acetic anhydride.

9. The process as claimed in claim 1, which comprises precipitating the derivative directly in the form of a scarcely soluble salt of a heavy metal, preferably the zinc salt.

10. The process as claimed in claim 2, which comprises precipitating the derivative from the organic phase by direct crystallization.

11. The process as claimed in claim 2, which comprises precipitating the derivative from the organic phase in the form of a salt by means of an alkylene diamine or an aromatic diamine.

12. The process as claimed in claim 11, which comprises using an optionally substituted alkylene diamine of the formula

$$NH_2—(CH_2)_n—NH_2$$

in which *n* is an integer of from 4 to 12, preferably 5 to 7.

13. The process as claimed in claim 12, which comprises substituting a hydrogen atom at the nitrogen atom and/or one or more hydrogen atoms of the methylene group by low molecular alkyl or aryl.

14. The process as claimed in claims 11 to 13, which comprises precipitating the derivative from the organic phase in the form of a 1,6-diaminohexane salt.

15. The process as claimed in claim 2, which comprises the solvent not completely miscible with water being n-butanol, isobutanol, ethyl acetate or methylisobutylketone.